# EUROPEAN PATENT SPECIFICATION

(11) **EP 3 897 489 B1**
(45) Date of publication and mention of the grant of the patent: **29.11.2023**
(21) Application number: 19836901.9
(22) Date of filing: 17.12.2019
(51) Int. Cl.: A61F 13/00, A61F 13/42

(54) **FLUID STATUS INDICATOR FOR A WOUND DRESSING**
FLÜSSIGKEITSSTANDANZEIGER FÜR WUNDVERBAND
INDICATEUR D'ÉTAT DE FLUIDE POUR UN PANSEMENT

(30) Priority: 21.12.2018 US 201862784187 P
(43) Date of publication of application: 27.10.2021
(73) Proprietor: 3M Innovative Properties Company, St. Paul, MN 55133-3427 (US)
(72) Inventor: LOCKE, Christopher B., San Antonio, Texas 78265 (US); ROBINSON, Timothy M., San Antonio, Texas 78265 (US)
(74) Representative: Simmons & Simmons
(86) International application number: PCT/US2019/066782
(87) International publication number: WO 2020/131833

(56) References cited:
- WO-A1-2012/171922
- WO-A1-2018/031932
- WO-A2-2014/108682
- US-A1- 2014 088 534

## Description

### BACKGROUND

The present disclosure relates generally to an indicator for a wound dressing configured to monitor a fluid level status of the wound dressing and/or the tissue to which the wound dressing is attached.

Typical moisture level indicating devices are configured to transition between an initial visual state (e.g., in which an indicia provided on the indicating device is not visible) to a second visual state (e.g., in which the indicia is visible) upon exposure to a predetermined level of fluid. However, such indicating devices fail to reset following the initial exposure to a predetermined amount of moisture. Thus, the indicating device will continuously be defined by the second visual state following the initial (and only) transition of the indicating device between the first visual state and the second visual state-even if moisture levels decrease to below the predetermined moisture level.

Monitoring moisture levels at or around a wound during wound treatment may be desirable for a variety of reasons. For example, monitoring the fluid capacity of an absorbent wound dressing may alert a user as to when the absorbent wound dressing is nearing it maximum fluid capacity, and thus may be used to prevent leakage, detachment, or rupture of the wound dressing that would otherwise occur it the oversaturation of the wound dressing were to go unnoticed.

Absorbent wound dressings are often characterized by variable fluid handling capacity (provided by, e.g., a drape having a high MVTR and/or a negative pressure wound therapy system). Thus, even upon nearing a maximum absorbent capacity, the evaporation of fluid through the drape layer 110 and/or evacuation of fluid via the negative pressure wound therapy system may increase the fluid capacity of the wound dressing. This reduction of the fluid level to below the predetermined threshold may obviate the need to replace the wound dressing, prolonging the duration during which the wound dressing can be used to treat a wound. An indicator capable of repeatedly and reversibly transitioning responsive to variations in fluid level that may occur over the course of use of such variable fluid handling capacity wound dressings would advantageously allow a user to track such changes as they occur. This would provide the user with reassurance that the wound dressing is functioning as intended, and would reduce the likelihood of the user prematurely replacing the wound dressing.

As another example, the extended presence of fluid between a wound dressing and a periwound surrounding a wound site can cause maceration or reduce adhesion of a wound dressing to a patient. Accordingly, monitoring fluid levels at the periwound may alert a user to situations in which moisture has accumulated in excess of a predetermined maximum acceptable level, and thus may be used to prevent complications such as reduced wound health and slowed wound healing.

The risk of maceration of skin is a composite of fluid presence and time. A presence on the skin of a lot of fluid for a short period of time will not necessarily result in damage, whereas the presence of a small amount for a very long period of time may negatively affect the wound. Given that even a small amount of moisture at the periwound may pose, the amount of moisture to which a wound dressing may be exposed during, or prior to, application to a patient (e.g. moisture along the skin of the patient, moisture in the ambient air, etc.) may exceed a maximum acceptable periwound fluid level threshold. An indicator capable of resetting following exposure to fluid in excess of a predetermined threshold would advantageously allow the indicator to be used to monitor periwound fluid levels, even if the indicator had been exposed to moisture levels prior to being attached along the periwound.

WO 2014/108682 discloses a wound dressing with an indicator.

### SUMMARY

The invention is defined in the appended claims, in which there is required an indicator for detecting a fluid status of a wound dressing, the indicator comprising: a lower layer; a marking provided along the lower layer; and a top layer; wherein in a first state, the marking is blocked by the top layer and not visible to a user under ambient or natural light; wherein in a second state, the marking is visible through the top layer under ambient or natural light; wherein in a third state, the marking is blocked by the top layer and not visible to a user under ambient or natural light, the third state occurring at a point in time subsequent to the second state; wherein the top layer is formed from a porous, hydrophilic structure defined by a plurality of fibers; and wherein, in the first state, the fibers have a first refractive index and in the second state the fibers have a second refractive index that is greater than the first refractive index.

A selection of optional features is set out in the dependent claims.

### BRIEF DESCRIPTION OF THE DRAWINGS

FIG. 1 illustrates a wound dressing provided with an indicator upon initial application to a wound site, according to an exemplary embodiment.
FIG. 2 illustrates an exploded view of an indicator, according to an exemplary embodiment.
FIG. 3A illustrates a top perspective view of an indicator, according to an exemplary embodiment.
FIG. 3B illustrates a bottom perspective view of the indicator of FIG. 3A.
FIG. 4A illustrates a wound dressing incorporating an indicator, according to an exemplary embodiment.
FIG. 4B illustrates a wound dressing incorporating an indicator, according to an exemplary embodiment.
FIG. 4C illustrates a wound dressing incorporating an indicator, according to an exemplary embodiment.
FIG. 5A illustrates the wound dressing of FIG. 4A upon the exposure to the indicator to a fluid, according to an exemplary embodiment
FIG. 5B illustrates the wound dressing of FIG. 4A upon the exposure to the indicator to a fluid, according to an exemplary embodiment
FIG. 5C illustrates the wound dressing of FIG. 4B upon the exposure to the indicator to a fluid, according to an exemplary embodiment

### DETAILED DESCRIPTION

Before turning to the figures, which illustrate certain exemplary embodiments in detail, it should be understood that the present disclosure is not limited to the details or methodology set forth in the description or illustrated in the figures. It should also be understood that the terminology used herein is for the purpose of description only and should not be regarded as limiting.

Referring generally to the FIGURES, an indicator configured to provide near real-time, dynamic indication of the fluid status (e.g., fill level, fluid absorbency capacity, etc.) of an article is described according to various embodiments.

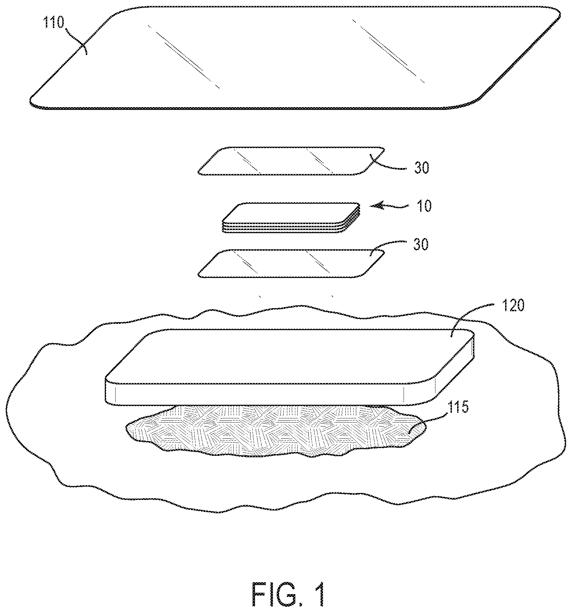

As illustrated in FIG. 1, an indicator 10 as described herein is advantageously used to monitor one or more different types of fluid statuses associated with the treatment of a wound 115. Additionally described with reference to the FIGURES are various embodiments of wound dressings incorporating such reversible, dynamic indicators.

### Indicator

In contrast to typical fluid indicating devices-which are only able to provide a one-time, irreversible transition from a first state to a second state-an indicator 10 as described according to any embodiment herein is configured to repeatedly and reversibly transition between a first state corresponding to a first fluid status of a wound dressing 100 (or any other absorbent article and/or article to which the indicator 10 may be attached), and a second state corresponding to a second fluid status of the wound dressing 100 responsive to changes in the fluid status of the wound dressing 100 over the course of its use.

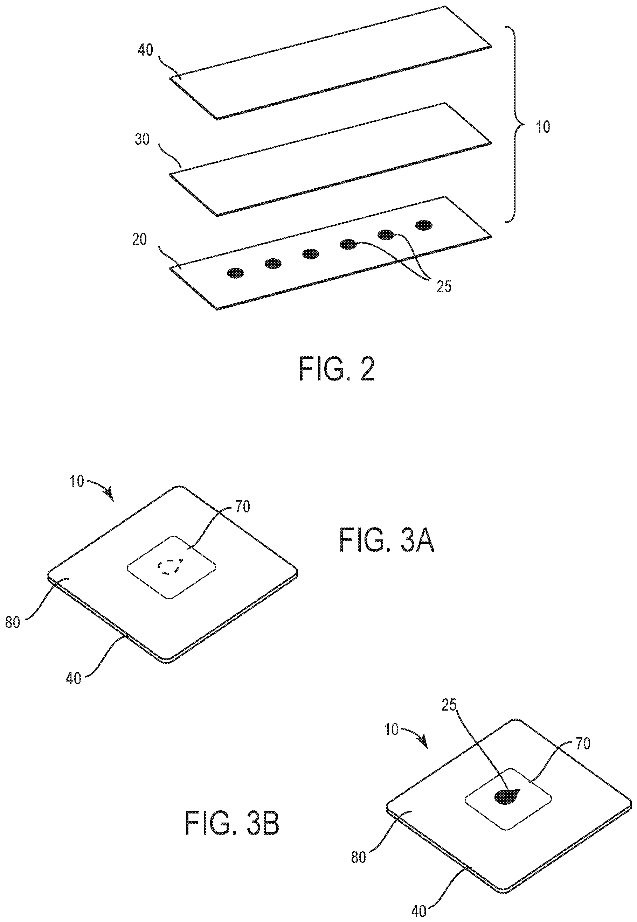

Referring to FIG. 2, the indicator 10 generally includes an indicator layer 20 and a top layer 40. As will be discussed in more detail below, the top layer 40 of the indicator 10 is configured to transition between a significantly (e.g. entirely) opaque state (e.g., a first state, a dry state) and a significantly (e.g. entirely) transparent state (e.g., a second state, a wet state) in response to the exposure of the top layer 40 to fluid. The indicator layer 20 includes one or more markings 25 that are selectively visible (i.e. capable of being viewed, unaided, by a human eye under ambient or natural light conditions) through the top layer 40, and is optionally also configured to wick fluid from the absorbent layer 120 of the wound dressing 100 (and/or any other surface atop which the indicator 10 is disposed) to facilitate the exposure of the top layer 40 to fluid. The indicator 10 optionally also includes one or more attachment layers 30 configured to secure the indicator 10 relative to the wound dressing 100 and/or to secure the indicator layer 20 relative to the top layer 40.

The transition of the top layer 40 from the opaque state to the transparent state-which causes one or more markings 25 provided along the indicator layer 20 to be made visible to a user-is configured to signal to a user that an article to which the indicator 10 is attached (e.g., an absorbent layer 120, or other layer, of a wound dressing 100) has reach a predetermined fluid status (e.g., a predetermined absorbent capacity of the absorbent layer 120 has been reached, the article has been exposed to a predetermined amount of fluid, etc.). As fluid is removed (e.g. suctioned, evacuated, evaporated, etc.) from the wound dressing 100 and the fluid status of the wound dressing 100 falls below the predetermined fluid status threshold, the indicator 10 transitions from the second state to the first state, causing the top layer 40 to revert to the opaque state in which the markings 25 of the indicator layer 20 are blocked from view by the top layer 40.

The ability of the top layer 40 to repeatedly transition between the opaque state and the transparent state provides a dynamic, two-way (i.e. reversible), near real-time indication via which any number of changes in the fluid status of the wound dressing 100 (or other article with which the indicator 10 is used) that occur over the course of the use of the wound dressing 100 may be communicated to a user. In contrast, typical fluid indicating devices allow only for only a one-time, irreversible transition from an initial state (representative of a first fluid state of a wound dressing) to a final state, (representative of a second fluid state of a wound dressing) responsive to the exposure of the wound dressing to a predetermined amount of fluid.

### a. Top Layer

The top layer 40 of the indicator 10 may be formed from any number of, or combination of, materials that are significantly opaque (e.g. entirely opaque) when dry (or substantially dry) and which are significantly transparent (e.g. entirely transparent, translucent, etc.) when exposed to fluid (e.g., when wet). The one or more materials forming the top layer 40 are configured to allow the top layer 40 to exhibit some degree of wicking in a lateral and optionally horizontal direction, such that fluid may be distributed across the top layer 40 when the top layer 40 is exposed to fluid from the indicator layer 20 (e.g., via the optional attachment layer 30).

The material from which the top layer 40 is formed is advantageously selected to have a high refractive index in a dry state (e.g., a first state, an opaque state) and to have a refractive index that is as close to the refractive index of the fluid (e.g. water) to which the top layer 40 is exposed when in a wet state (e.g. a second state, a transparent state), so as to increase the opacity of the top layer 40 in the dry state and increase the transparency of the top layer 40 in the wet state. According to various embodiments, the refractive index of the top layer 40 in the wet state is between approximately 1.0 and approximately 1.5, more specifically between approximately 1.25 and 1.4, and even more specifically approximately 1.33. Non-limiting examples of materials that form the top layer 40 include, e.g., polyvinylidene difluoride (PVDF); DURAPORE 0.2 micron filter paper; or any other number of flexible, breathable materials configured to change from opaque to transparent when exposed to fluid.

The materials used to form the top layer 40 are defined by a porous hydrophilic structure formed of fibers that define a plurality of air-filled spaces in the dry state of the indicator 10. When the indicator 10 is in the dry state, the difference between the refractive index of the fibers and the refractive index of the air within the spaces causes light to be dispersed, resulting in the top layer 40 appearing opaque. As the top layer 40 is exposed to fluid (i.e. as the indicator 10 transitions from the dry state to the wet state), the air filling the spaces between the fibers is replaced with fluid. Because the refractive index of the fluid is greater than the refractive index of air, this replacement of air in the spaces with fluid results in less light being dispersed by the top layer 40 when the indicator 10 is in the wet state, thus increasing the transparency of the top layer 40.

The fibers of the top layer 40 are optionally formed from a material defined by a high refractive index, similar to that of the fluid to which the top layer 40 will be absorbed, so as to increase the opacity of the top layer 40 when the indicator 10 is in the dry state and to increase the transparency of the top layer 40 when the indicator 10 is exposed to fluid (i.e., in the wet state). In some embodiments, the fibers are formed from a material defined by a refractive index that is substantially the same as the refractive index of water.

Some or all of the fibers forming the porous structure are optionally formed of a material configured to absorb the fluid to which the top layer 40 is exposed when the indicator 10 is in the wet state. In addition to the decrease in dispersion resulting from the wicking of fluid into the air gaps as the top layer 40 is exposed to fluids-the dispersion of light in the wet state is in such embodiments in further reduced as a result of the absorption of fluid by the fibers. In particular, the absorption of fluid by the absorbent fibers also acts to bring the refractive index of the fibers closer to that of the fluid, and thereby further increases the apparent transparency of the top layer 40. The fibers may be as fine as possible, so as to increase the amount of fluid that may be absorbed by the fibers, and thus further minimize the difference between the refractive indices of the fibers and the fluid.

In view of the effect that the absorption of fluid has on minimizing the difference between the refractive index of the fiber and that of the fluid in the wet state, the absorbent fibers are optionally formed from a material having a higher refractive index in the dry state than that of a material that would be selected for fibers formed from a non-absorbent material. Accordingly, in addition to exhibiting an increased transparency when exposed to fluid, in such embodiments in which some or all of the fibers forming the top layer 40 are absorbent, the top layer 40 may additionally exhibit an increased opacity in the dry state..

In some embodiments, the absorbent fibers may comprise a superabsorbent polymer ("SAP") and/or other material having increased fluid absorbing capabilities, such as, but not limited to, e.g., sodium polyacrylate, polyacrylamide copolymer, ethylene maleic anhydride copolymer, cross-linked carboxymethyl cellulose, polyvinyl alcohol copolymers, cross-linked polyethylene oxide, etc. The increased ability of fibers made from such a SAP to absorb fluid may further minimize the difference between the refractive indices of the fibers and the fluid to which the top layer 40 is exposed, thus further increasing the transparency of the top layer 40 in the wet state.

According to some embodiments, instead of, or in addition to, the material used to define top layer 40 comprising a porous, fibrous, hydrophilic structure, the top layer 40 is optionally defined by a layer of polymer particles that have been sprayed directly or indirectly onto the indicator layer 20. Similar to the increased transparency resulting replacement of air with fluid within the gaps between adjacent fibers that occurs upon the exposure of the fiber-formed top layer 40 (described above) to a fluid, the replacement of air with fluid within the gaps defined between adjacent polymer particles that have settled on the indicator layer 20 also increases the transparency of a polymer particle-formed top layer 40 responsive to the exposure of the indicator 10 to fluid. In some embodiments, the polymer particles may optionally be formed from an absorbent material to further increase the opacity and transparency of the polymer-particle formed top layer 40 in the dry state and the wet state, respectively, in a manner similar to that described with reference to the absorbent fiber embodiments discussed above.

### b. Indicator Layer

The indicator layer 20 may be formed of any number of hydrophilic, porous materials capable of acquiring and distributing (e.g. wicking) fluid from the absorbent layer 120 (or other structure to which a lower surface of the indicator 10 is attached) to the top layer 40. Non-limiting examples of materials from which the indicator layer 20 may be formed include low refractive index cellulose; a WHATMAN^{®} nylon filter member with 0.45 micron perforations; blotting paper, such as, e.g., blotting paper which is split and optionally welded on one or both sides to a fusible fiber (such as, e.g., that used for the attachment layer 30); etc.

According to various embodiments, the material forming the indicator layer 20 (and/or one or both of the top layer 40 and attachment layer 30) is optionally configured to prevent, or minimize, the impact of the exposure of the indicator 10 to a colored (i.e. not clear) fluid or exudate on the visual appearance of the indicator 10 (e.g., to prevent discoloration of the indicator 10, the disguising of or other interference with the visibility of the markings 25 printed on the indicator layer 20, etc.). Alternatively, or additionally, the indicator 10 may include an optional filter layer configured to prevent the discoloration of, or other effect on the visual appearance of, the indicator 10.

Referring to FIG. 2, one or more markings 25 that become visible upon the exposure of the top layer 40 to fluid are formed along the indicator layer 20. Advantageously, the size and color of the markings 25 are selected to provide a high degree of contrast with the rest of the indicator layer 20 to increase the visibility of the markings 25 when the top layer 40 becomes transparent (i.e. in the wet state). These one or more markings 25 provided along the indicator layer 20 may comprise any number of, or combination of, e.g., text, numbers, images, icons, symbols, logos, etc. or other indicia. The one or more markings 25 may be of any size and color.

The one or more markings 25 are formed along the upper surface of the indicator layer 20 using any number of known materials (e.g. inks, dyes, etc.). The material used to form the markings 25 is advantageously configured to remain fixed relative to the material of the indicator layer 20, so as to prevent the markings 25 from bleeding or otherwise being mobile when exposed to fluids. According to some embodiments, the material used for the markings 25 is biocompatible, hydrophobic, and non-soluble when exposed to water or other fluids. In some embodiments, the material used for the markings 25 may have a gel-like viscosity, so as to minimize or prevent the movement of the markings 25 relative to the indicator layer 20. The markings 25 may be provided along the indicator layer 20 according to any number of methods such as, e.g., by spraying the material particles onto the indicator layer 20 upper surface, screen printing, etc.

In some embodiments in which the indicator 10 is to be attached to (i.e. in direct contract with) an article (e.g. an absorbent layer 120 of a wound dressing 100, a manifolding layer 130 of a wound dressing 100, etc.) composed of a material (e.g. a foam) having a sufficiently high porosity (e.g. a porosity of approximately 100 pores per inch or more), the markings 25 are optionally provided along the top surface of the article (e.g. absorbent layer 120, manifolding layer 130, etc.), and the top layer 40 of the indicator 10 is directly or indirectly (e.g., via an attachment layer 30) attached to the article, such that the indicator layer 20 is defined by the article.

As illustrated in FIGS. 3A and 3B, in yet other embodiments, the indicator 10 is optionally defined by only a top layer 40. Instead of markings 25 being provided on a separate indicator layer 20 (such as, e.g., illustrated by the embodiment of FIG. 2), as shown in FIG. 3B, the markings 25 are instead provided directly along a lower surface of the top layer 40. Similar to the top layer 40 described with reference to the indicator 10 embodiment of FIG. 2, a central portion 70 of the single-layer indicator 10 of FIGS. 3A and 3B is formed of PVDF (or other material configured to transition from an opaque state to a transparent state upon exposure to a fluid). A border 80 formed of a wicking material (which may be the same material as that used for the central portion 70) surrounds and extends outwardly relative to the central portion 70. The wicking capabilities and added surface area provided by the border 80 expedite the exposure of the central portion 70 of the indicator 10 to fluids from the absorbent layer 120 (or other structure to which the indicator 10 is attached), and thus facilitate the transition of the indicator 10 to the transition to the transparent state in which the markings 25 upon the indicator 10 being exposed to a predetermined fluid level threshold.

In other embodiments not according to the invention, an indicator 10 comprising a single layer is optionally formed from multilayered fibers comprising a pigmented inner layer that becomes visible upon the exposure of the fibers to fluid. In such embodiments the fibers optionally comprise a core structural layer (which need not necessarily be hydrophilic, and can, e.g., be made from polyesters, polyamides, etc.). The inner, pigmented polymer layer surrounds (or optionally defines) the core structural layer. The pigmented layer need not be hydrophilic, and may comprise the same materials as those used to form the optionally provided core. An outer hydrophilic layer (formed, e.g., from a SAP) annularly surrounds the pigmented layer, and is configured to absorb water or other fluids to which the fiber is exposed. As the initially opaque outer hydrophilic layer absorbs water and wicks fluid into gaps defined between the fibers, the refractive index of the fibers is decreased, which increases the transparency of the outer layer and allows the inner pigmented layer to become visible.

### c. Attachment Layer

As illustrated in FIGS. 1 and 2, according to various embodiments, the indicator 10 optionally includes one or more attachment layers 30 that extend along some or all of the upper and/or lower surfaces of the top layer 40 and/or indicator layer 20, and which are used to secure the top layer 40 relative to the indicator layer 20 and/or to secure the indicator 10 relative to the wound dressing 100. For example, as illustrated by the indicator 10 embodiment of FIG. 2, an attachment layer 30 is provided between the lower surface of the top layer 40 and an upper surface of the indicator layer 20 to attach the top layer 40 and indicator layer 20 relative to one another. As the ability of the indicator 10 to indicate the fluid status of an article (e.g. a wound dressing 100) is dependent on the indicator 10 being exposed to fluids absorbed by and/or present along the article, the wound dressing 100 optionally includes an attachment layer 30 provided along a lower surface of the indicator 10 (e.g., along a lower surface of the indicator layer 20) which is configured to secure the indicator 10 in contact with an upper surface of the absorbent layer 120 (see, e.g., FIG. 1). To ensure the visibility of the indicator 10 upon the application of a wound dressing 100 to a patient, an optional attachment layer 30 is provided along an upper surface of the indicator 10 (e.g., along an upper surface of the top layer 40) to secure the indicator 10 relative to a lower surface of the drape layer 110.

As will be understood, in embodiments in which more than one attachment layer 30 is used, some or all of the attachment layers 30 may be defined by the same or different structures, compositions, dimensions, etc. The attachment layer(s) 30 incorporated/used with the indicator 10 may be formed from any number of different materials and may comprise any number of different structures that extend along all of or a portion of the upper and/or lower surface of the top layer 40 and/or indicator layer 20. According to various embodiments, the attachment layer(s) 30 comprises a hot-melt fusible fiber that may be heated to attach the desired layers of the indicator 10 and/or wound dressing 100 to one another. According to various embodiments, and in particular in embodiments in which an attachment layer 30 is used to attach the top layer 40 of the indicator 10 to one or both of the indicator layer 20 and the bottom surface of the drape layer 110, the melt range of the fusible fiber is sufficiently low so as to prevent the heat from melting or otherwise affecting the top layer 40 in such a manner that would result in the top layer 40 becoming permanently transparent.

According to other embodiments, the attachment layer 30 may comprise an adhesive, such as, e.g. an adhesive printed and/or sprayed on all of or a portion of the upper and/or lower surface of the top layer 40 and/or indicator layer 20. In some such embodiments, the adhesive may additionally or alternatively be printed or otherwise provided on the top surface of the absorbent layer 120 (or other dressing structure to which the indicator 10 is to be attached) and/or the lower surface of the drape layer 110 of the wound dressing 100.

### Indicator Integration with a wound dressing

As discussed above, an indicator 10 as described herein may advantageously be used to monitor one or more types of fluid statuses associated with the use of a variety of different wound dressings 100. The wound dressings 100 with which the indicator 10 may be used may include wound dressings 100 configured for use in any number of different types of wound treatments, and may accordingly be defined by a variety of different structures, components, modes of operation, etc. In addition to being configured to be used with standalone wound dressings 100, the indicator 10 may also be used with wound dressings 100 that form a part of a larger wound therapy system such as, e.g., a negative pressure wound therapy system ("NPWT system").

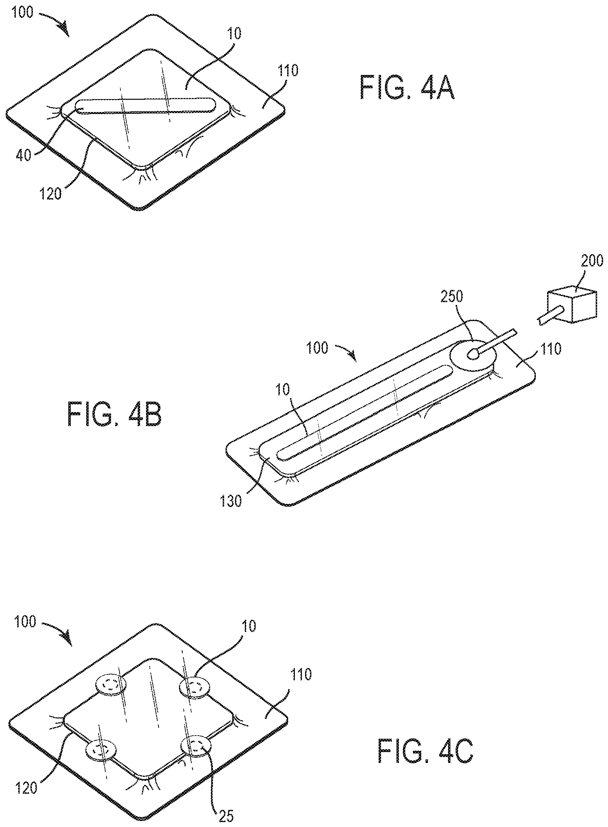

As shown in FIGS. 4A and 4C, according to various embodiments, the wound dressing 100 is an absorbent wound dressing, and generally includes an absorbent layer 120 and a drape layer 110. The absorbent layer 120 of the wound dressing 100 is adapted to collect fluid from the wound 115, and may be formed from any material that is adapted to receive and store fluids. For example, without limitation, the absorbent layer 120 may be formed from one or more materials such as a capillary-containing material,
super absorbent fiber/particulates, hydrofiber, sodium carboxymethyl cellulose, alginates, sodium polyacrylate, etc.

The drape layer 110 is configured to support the absorbent layer 120 (and any optionally included additional wound dressing 100 layers) relative to the wound 115, and is also optionally configured to secure the wound dressing 100 to the patient (e.g., via an adhesive provided along an outer perimeter of the lower surface thereof). Any material or combination of materials may be used for the drape layer 110. In some embodiments, the drape layer 110 is a thin layer of polyurethane film, such as, e.g., ESTANE 5714F. Other suitable polymers for forming drape layer 110 include polyalkoxyalkyl acrylates and methacrylates, such as those described in Great Britain Patent Application No. 1280631A filed November 22, 2002.

In some embodiments, the fluid absorbency capacity of the absorbent wound dressing 100 remains unchanged over time (i.e. is static). Once the fluid capacity of the wound dressing 100 has been reached, the wound dressing 100 will be incapable of absorbing or holding any additional fluid, such that the exposure of the wound dressing 100 to any additional fluid puts the wound dressing 100 at a risk of macerating, leaking, swelling and becoming detached, etc.

In other embodiments, the drape layer 110 of the standalone absorbent wound dressing 100 into which the indicator 10 is incorporated advantageously is defined by a high moisture vapor transmission rate ("MVTR"), such that the drape layer 110 is substantially impermeable to liquid and substantially permeable to moisture vapor. In other words, drape layer 110 is permeable to water vapor, but not permeable to liquid water or wound exudate. This ability for fluid within the wound dressing 100 to evaporate via the drape layer 110 may result in the total fluid handling capacity (TFHC) of wound dressing 100 varying one or more times between a dry state and a wet state over the course of the use of the wound dressing 100.

As representatively illustrated by FIG. 4B, according to some embodiments, the indicator 10 may be used with a wound dressing 100 that is used with a NPWT system 200. The application of negative pressure to the wound dressing 100 during treatment of the wound 115 may result in the evacuation of some or all of the fluid present within the wound dressing 100 (e.g., fluid exuded by wound 115, fluid instilled to the wound 115 as part of instillation therapy, etc.), thus resulting in the TFHC of the wound dressing 100 varying over the course of wound treatment. In embodiments in which the drape layer 110 of the wound dressing 100 used with the NPWT system 200 is defined by a high MVTR, the evaporation of fluids from within the wound dressing 100 may result in further fluctuations in the fluid status of the wound dressing 100 during use. Notably, the wound dressing 100 in such embodiments may include a manifolding layer 130 (e.g. a reticulated, open-cell polyurethane or polyether foam) configured to transmit a vacuum to the wound 115) and/or may include a variety of other layers, such as, e.g., an absorbent layer 120. In yet other embodiments, the wound dressing 100 with which the indicator 10 is used is optionally used in conjunction with an instillation therapy wound treatment system (e.g., a combined negative pressure/instillation therapy system) in which predetermined quantities of a fluid are instilled into the treatment space defined between the drape layer 110 and wound 115 for predetermined durations of time..

Non-limiting examples of wound dressings 100 into which the indicator 10 described herein may be incorporated include, but are not limited to, a NANOVA^{™} wound dressing 100 available from Kinetic Concepts, Inc., of San Antonio; a TIELLE^{™} wound dressing 100 also available from Kinetic Concepts, Inc., a PICO wound dressing 100 available from Smith & Nephew, of the United Kingdom; an AQUACEL^{®} wound dressing available from ConvaTec, of the United Kingdom; an AVELLE^{™} wound dressing, also available from ConvaTec; a GranuFoam^{®} dressing available from Kinetic Concepts, Inc.; a V.A.C. VeraFlo^{®} foam, also available from Kinetic Concepts, Inc.; etc.

During integration of an indicator 10 into a wound dressing 100, the indicator 10 is advantageously located directly underneath the drape layer 110 (i.e., without any other component of the wound dressing 100 interposed therebetween) such the visibility of the indicator 10 to a viewer (e.g., the patient, a medical provider, an optical reader, etc.) is not obscured. An attachment layer 30 (e.g., an adhesive fusible fiber, etc.) optionally secures an upper surface of the indicator 10 relative to a lower surface of the drape layer 110 to prevent inadvertent movement of the indicator 10.

The transition of an indicator 10 incorporated into a wound dressing 100 between the dry state and the wet state (and vice versa) may be used to indicate to a user a variety of different types of changes in fluid status during the use of the wound dressing 100. The significance of the transition of the indicator 10 may vary based on a variety of factors such as, e.g., the type of wound dressing 100 being used, the type of treatment being provided, the type of fluid status change that is being monitored, etc. Accordingly, the manner in which the indicator 10 is integrated into a wound dressing 100 may be varied as desired to allow a user to observe and be alerted to a variety of different types fluid status changes during the treatment of a wound 115.

For example, as illustrated in FIGS. 4A and 4C, in embodiments in which the wound dressing 100 comprises an absorbent layer 120, the indicator 10 is optionally integrated into the wound dressing 100 such that a lower surface of the indicator 10 is positioned at least partially in direct contact with the absorbent layer 120, allowing the indicator 10 to monitor the remaining fluid capacity of the absorbent layer 120. In some such embodiments, the indicator 10 may be supported relative to the absorbent layer 120 via an attachment of the indicator 10 (e.g., via attachment layer 30) to one or both of an upper surface of the absorbent layer 120 and/or a lower surface of the drape layer 110.

In embodiments in which the absorbent wound dressing 100 is defined by a dynamic fluid capacity (provided by, e.g., a drape layer 110 having and high MVTR and/or the use of the absorbent wound dressing 100 with a NPWT system), the indicator 10 may subsequently revert to the opaque dry state once sufficient fluid from the absorbent layer 120 has been evaporated or otherwise removed, such that the fluid collected within the absorbent layer 120 decreases to below the predetermined fluid status transition threshold. The indicator 10 may subsequently revert between the dry state and the wet state any number of additional times over the course of use of the wound dressing 100 responsive to any additional changes in the fluid level of the wound dressing 100. Notably, in embodiments in which the absorbent wound dressing 100 has a static fluid capacity and is used as a stand-alone wound treatment, the static nature of the fluid absorbency capacity of the wound dressing 100 will result in only a single transition of the indicator 10 from the first state to the second during the use of the wound dressing 100-despite the indicator 10 being configured to allow for a reversible transition between first and second indicating states.

Referring to FIG. 4C, in embodiments in which it is desired to monitor moisture levels at the periwound, the indicator 10 is integrated into the wound dressing 100 at a location along the wound dressing 100 that overlies (or is adjacent) the periwound. For example, the indicator 10 is optionally integrated at a location along a border defined by and extending around an outer perimeter of the drape layer 110; adjacent, or at, an interface between the border and an absorbent layer 120, a manifolding layer 130, or other wound dressing 100 layer; etc. In such embodiments, the indicator 10 may be supported relative to the wound dressing 100 such that the lower surface of the indicator 10 is positioned directly in contact with the periwound, or may be positioned in direct contact with an upper surface of an optional wound interface layer or other component of the wound dressing 100 that extends between the drape layer 110 and the periwound.

Referring to FIG. 4B, in embodiments in which the wound dressing 100 defines a component of a NPWT system 200 (or a component of any other additional wound treatment system, such as, e.g., an instillation therapy system, etc.), it may be desired to maintain a particular moisture level within a treatment space defined beneath the wound dressing 100. For example, during NPWT therapy using the NPWT system 200 it may be desired to maintain a moisture level within the wound dressing 100 below a predetermined threshold level, whereas during instillation therapy it may desired to maintain a moisture level within the wound dressing 100 above a predetermined threshold level. As illustrated in FIG. 4B, in various such embodiments the indicator 10 is supported within the wound dressing 100 such that a lower surface is in direct contact with a manifolding layer 130 of the wound dressing 100 (and/or other component of the wound dressing 100) and/or with the wound 115. In embodiments in which the additional wound treatment system includes a treatment device (e.g. an instillation pump, a negative pressure source, etc.) that is selectively operable to effect a change in the moisture level within the treatment space, an optical reader optionally incorporated into the wound treatment system can be used to automatically control the operation of the treatment device responsive to the detection of a transition of the indicator 10 between the dry state and the wet state (and/or vice versa).

According to various embodiments, the indicator 10 is optionally used to verify the operation of a wound treatment system with which the wound dressing 100 is used. For example, even under proper operating conditions, changes in the total fluid handling capacity of the wound dressing 100 that occur fluid as evaporated, evacuated, or otherwise removed from the wound dressing 100 and/or as wound exudate is collected by the wound dressing 100 may not result in any noticeable, visible changes to the overall dimensions, shape or other aspects of the visual appearance of the wound dressing 100 (such as, e.g., a swelling of the wound dressing 100 during instillation of fluid, a contraction of the wound dressing 100 as fluid is evaporated or otherwise removed from the system, etc.). The incorporation of a dynamic, reversible indicator 10 capable of providing a visibly distinguishable transition in response to such changes would thus allow a user to visually verify that the wound dressing 100 and wound treatment system are functioning as intended.

In some embodiments, the incorporation of an indicator 10 can also optionally be used to verify a proper attachment of the wound dressing 100 to the patient and/or to verify a proper fluid-tight seal between components of an optional wound therapy device with which the wound dressing 100 is used. For example, as the presence of fluid at locations such as, e.g., between a lower surface of the drape layer 110 border and the skin of the patient; between a port connector 250 and an upper surface of the drape layer 110 to which the port connector 250 is sealed (see, e.g. FIG. 4B), etc. often results from an improper sealing attachment between elements, the incorporation of an indicator 10 at such locations may be used to alert a user of a leak which may need to be repaired.

In yet other embodiments, the incorporation of indicators 10 into a wound dressing 100 is used to allow a user to monitor the progression of wound healing. For example, the use of multiple indicators 10 that are each configured to transition at different predetermined fluid level thresholds may be used to track how quickly moisture levels at the wound 115 are decreasing. In some such embodiments, upon an initial application of the wound dressing 100 to a highly exuding wound 115, markings 25 on each of the multiple indicators 10 will initially be visible. As the wound 115 heals and the amount of exudate decreases, the reduced moisture level will result in less sensitive indicators 10 being reset to the dry state. Thus, by tracking which indicators 10 remain in the wet state, a user may estimate the healing progression of the wound 115.

The predetermined fluid level at which it is desired that the indicator 10 transition between the first state and the second state (and vice versa) may vary depending on the type of fluid status that is being monitored. For example, in embodiments in which the indicator 10 is used to alert a user to an absorbent layer 120 nearing its maximum absorbency capacity, the fluid level at which the indicator 10 transition between the first state and the second state may be selected to be relatively high, such that the indicator 10 transitions responsive to the fluid level of the wound dressing 100 nearing the maximum fluid capacity of the absorbent layer 120. Given that the presence of even a minimal amount of fluid along the periwound may have undesirable effects on wound healing, in another exemplary embodiment, the indicator 10 may be configured to transition between the first state and the second state responsive to the exposure of the indicator 10 to a relatively low fluid level corresponding to a maximum allowable periwound moisture level. In yet other embodiments (such as, e.g., described with reference to the use of indicators 10 to monitor wound healing progression) it may be desired to provide a user with a more granular ability to monitor fluid levels, with the indicators 10 that are incorporated into the wound dressing 100 being configured to transition between the first state and the second state responsive to varying fluid levels.

The sensitivity of an indicator 10 (e.g. the rate at which an indicator 10 transitions between the dry state and wet state, and vice versa) may vary based on factors such as, e.g., the selection of and relative dimensions of the materials defining the indicator 10, the volume defined by the wound dressing 100 into which the indicator 10 is incorporated, etc. An indicator 10 defined by a higher sensitivity will transition at a lower fluid level threshold than a less sensitive indicator 10. Depending on the desired indicator 10 transition threshold, the sensitivity of the indicator 10 may be increased by selecting materials for the indicator layer 20 having a high degree of horizontal and/or vertical wicking, or which otherwise expedite the uptake of fluid. The sensitivity of an indicator 10 may also be increased by selecting a top layer 40 material having a high refractive index when wet (e.g. a material having a high absorbency), which increases the rate at which the top layer 40 becomes transparent upon exposure to fluid. Varying the thickness of the indicator layer 20 and /or top layer 40 and/or modifying the amount of adhesive (or other material) used to define the attachment layer 30 may also be used to either impede or expedite the uptake of fluid to achieve a desired indicator 10 sensitivity. In various embodiments, varying the MVTR defining the drape layer 110 of the wound dressing 100 into which the indicator 10 is incorporated also optionally allows a user to vary the rate at which the indicator 10 will reset to the dry state following the exposure of the indicator 10 to fluid, with the resetting of the indicator 10 used in a wound dressing 100 with a drape layer 110 having a high MVTR occurring at a faster rate than the resetting of an indicator 10 used in a wound dressing 100 with a drape layer 110 defined by a low MVTR.

The unintentional exposure of the upper surface of the top layer 40 and/or sides of the indicator 10 to fluid may unintentionally trigger a transition of the indicator 10 to the wet state (in which the markings 25 are visible) that is not representative of the fluid level which the indicator 10 is monitoring. Accordingly, in various embodiments, portions (e.g. the entirety) of the sides and/or upper surface of the indicator 10 are sheathed (e.g. surrounded) in a cover layer (e.g. a fluid impermeable film and/or coating). The cover layer is configured to prevent the exposure of the indicator 10 to fluids that have been horizontally wicked from portions of the wound dressing 100 and/or wound 115 that extend radially outward from the location at which the fluid level is being monitored and/or to prevent the exposure of the indicator 10 to fluids that have condensed on the drape layer 110 and dripped onto an upper surface of the indicator 10. The portion of the cover layer extending along the upper surface of the indicator 10 (and optionally the other portions of the cover layer) are formed from a material defining an MVTR that does not impede the ability of the indicator 10 to evaporate fluid.

Advantageously, the cover layer may be formed from a material defining an MVTR that allows moisture from the indicator 10 to be evaporated at a rate substantially similar to that at which fluid is evaporated from the component of the wound dressing 100 and/or the portion of the wound 115 to which the indicator 10 is attached, such that resetting of the indicator 10 from the wet state to the dry state is representative of the decrease in moisture levels within the wound dressing 100 and/or at the wound 115. Similarly, in embodiments in which an attachment layer 30 secures an upper surface of the indicator 10 relative to the drape layer 110 (e.g., to ensure that the indicator 10 is visible to a view), the attachment layer 30 is advantageously configured to evaporate fluids at a rate that is the same (or substantially the same) at which moisture is evaporated from the component of the wound dressing 100 and/or portion of the wound 115 to which the lower surface of the indicator 10 is attached.

As illustrated in FIG. 4C, in various embodiments, the indicator 10 is incorporated into the wound dressing 100 along an adhesive-coated lower surface of the wound dressing 100 (e.g., at an outer periphery of the wound dressing 100 configured to be secured to the skin of a patient). In some such embodiments, the adhesive provided along the lower surface of the drape layer 110 may pose an impediment to the evaporation of fluids through the wound dressing 100. However-unless the adhesive on the drape layer 110 disproportionally effects evaporation from the indicator 10-the adhesive along the drape layer 110 is not removed prior to attaching the indicator 10 to the drape layer 110 adhesive, as the reduced rate of evaporation from the indicator 10 will be reflective of the rate at which moisture may be caused to accumulate along the skin. In embodiments in which an attachment layer 30 is used to secure a lower surface of the indicator 10 to a component of the wound dressing 100 and/or the wound 115, the attachment layer 30 is advantageously configured to not interfere with the ability of indicator layer 20 to absorb fluid from the wound dressing 100 component and/or wound 115.

The indicator 10 can be defined by a variety of different shapes and sizes (e.g. strips, dots, triangles, etc.) and/or other configurations (e.g., a ring-like structure configured to extend around a border region of the drape layer 110 of the wound dressing 100), and may vary based on a variety of different factors, such as, e.g., the shape and size of the wound dressing 100 with which the indicator 10 is to be used, the type of wound 115 that the wound dressing 100 is intended to be used with, the number of indicators 10 that are to be provided along the wound dressing 100, the intended viewer of the indicator 10 (e.g. a patient, a medical provider, an optical reader, etc.), etc. The number of indicators 10 incorporated into a wound dressing 100 and the number of markings 25 provided on the indicator 10 may also vary based on these factors.

For example, a user may be interested only in monitoring a single fluid level status (e.g., a moisture level at the periwound, the remaining fluid capacity of an absorbent layer 120, etc.) at a single location relative to the wound dressing 100 and/or wound 115. In such embodiments, it may be sufficient to integrate a single indicator 10 having a single marking 25 thereon into the wound dressing 100 at a single location relative to wound dressing 100. For example, in embodiments in which the wound dressing 100 is applied to a patient such that gravity causes a pooling of fluid along a bottom portion thereof (e.g., a wound dressing 100 applied to a leg or upper arm of a patient), it may be sufficient to monitor moisture levels at the periwound using a single indicator 10 integrated into the wound dressing 100 at a location along an outer perimeter of the drape layer 110 corresponding to a lower-most position of the wound dressing 100.

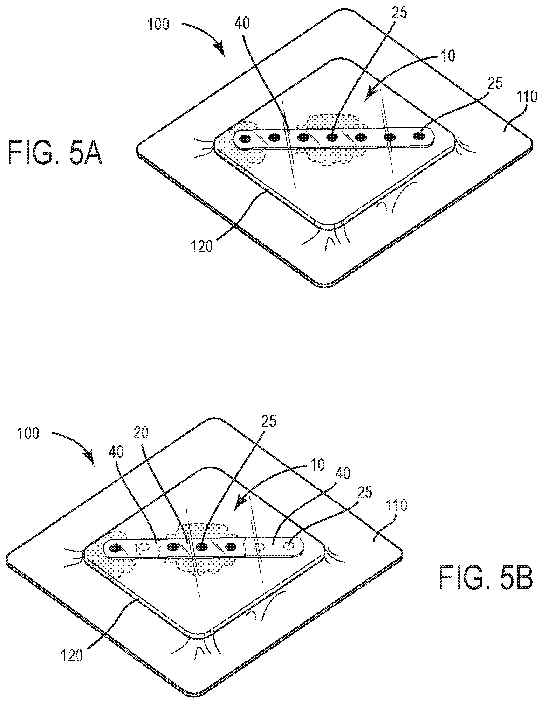

In other embodiments (e.g., when the wound dressing 100 extends horizontally, or substantially horizontally upon attachment to a patient), a user may be interested in monitoring a particular fluid level status (e.g., a moisture level at the periwound, the remaining fluid capacity of an absorbent layer 120, etc.) at a plurality of locations relative to the wound dressing 100. In some such embodiments, the user may be interested in being alerted to a fluid level reaching a predetermined threshold at any of the various locations, without needing to know at which specific location(s) the threshold fluid level was detected. For example, alerting a user to the detection of excess moisture at any one location along a periwound may be sufficient for a user, without needing to alert the user to the specific location at which the threshold fluid level was detected. Accordingly, one or more of the top layer 40, indicator layer 20 and/or attachment layer 30 in such embodiments are configured to provide a high degree of horizontal wicking. Accordingly, as representatively illustrated by FIG. 5A, the exposure of the indicator 10 to fluid at even a single location will result in fluid being dispersed across substantially all of (or the entirety of) the top layer 40, thereby triggering a transition of the indicator 10 to the transparent state along even those portions of the indicator 10 that extend outwardly beyond the location at which the indicator 10 was initially exposed to fluid.

Alternatively, a user may desire to be able to identify the specific area(s) relative to the wound dressing 100 at which the predetermined fluid threshold has been reached. Accordingly, as shown in FIG. 5B, in some embodiments, a single indicator 10 is configured such that the horizontal dispersion of fluids at locations extending between adjacent markings 25 is minimized, or prevented. In contrast to the embodiment described with reference to FIG. 5A, the exposure of the top layer 40 of the indicator 10 of FIG. 5B to fluid is limited to the portions of the top layer 40 lying near or directly above a portion of the indicator layer 20 that has been exposed to the predetermined fluid level threshold. Accordingly, as shown in FIG. 5B, in such embodiments, those markings 25 overlying portions of the indicator layer 20 that have not been exposed to the predetermined fluid level threshold will remain hidden from view.

The selective ability of only certain portions of the top layer 40 to be exposed to fluids of the embodiment of FIG. 5B may be achieved according to various arrangements. For example, materials defined by a low degrees of horizontal wicking can be used to form the indicator layer 20, attachment layer 30 and top layer 40 of the indicator 10. In other embodiments, portions of the indicator layer 20 (and optionally the top layer 40 and/or attachment layer 30) that extend between adjacent markings 25 are formed from a fluid impermeable, or substantially fluid impermeable, material.

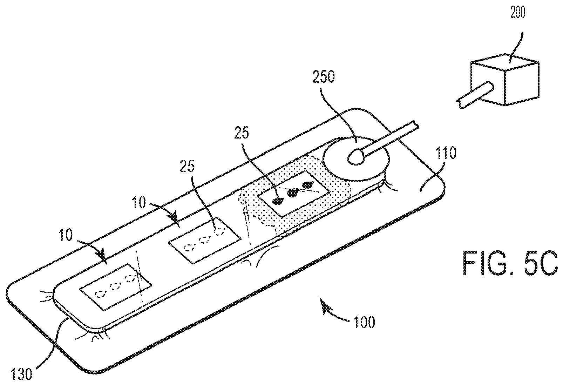

As representatively illustrated in FIG. 5C, as an alternative to (or in addition to) the use of a single indicator 10 such as described with reference to FIG. 5B (in which the indicator 10 is defined by a high specificity which allows for the selective transition of option certain portions of the indicator 10 to the wet state upon exposure to fluid), the monitoring of a particular type of fluid level at a plurality of location along the wound 115 and/or wound dressing 100 may be accomplished using a plurality of indicators 10 that are individually positionable at the various locations relative to the wound dressing 100.

In embodiments in which a plurality of indicators 10 are used to monitor a particular type of fluid status (e.g., a moisture level at the periwound, the remaining fluid capacity of an absorbent layer 120, a moisture level within the wound dressing 100, etc.) at a plurality of locations relative to the wound dressing 100 (e.g., as shown in FIG. 5C), the individual indicators 10 can be incorporated into the wound dressing 100 according to a number of different arrangements. For example, some or all of the indicators 10 are optionally defined as discrete structures that are provided separately from one another, and can individually be attached and integrated into the wound dressing 100 entirely independent of one another in any desired pattern, arrangement, spacing, etc.

In other embodiments, some or all of the plurality of indicators 10 are optionally interconnected to define an array that allows the plurality of indicators 10 to be simultaneously incorporated into the wound dressing 100 as a single unit. In such embodiments, the individual indicators 10 forming the array may be connected according to a variety of arrangements and configurations. For example, the array is optionally defined by a plurality of individual, discrete indicators 10 that are attached along an upper and/or lower surface of an attachment layer 30, which may subsequently be attached to a lower surface of the drape layer 110 and/or an upper surface of the absorbent layer 120 (or other layer of the wound dressing 100). In other embodiments, one or more (e.g. all) of the indicator(s) 10 are optionally provided pre-attached directly to one or more components of the wound dressing 100 (e.g., on the lower surface of the drape layer 110, on a top of an absorbent layer 120, along a manifolding layer 130, etc.), obviating the need to integrate the indicators 10 into the wound dressing 100 during the application of the wound dressing 100 to a patient.

In various embodiments, the individual indicators 10 defining the plurality of indicators 10 that are integrated into the wound dressing 100 to monitor a particular type of fluid status (e.g., .g., a moisture level at the periwound, the remaining fluid capacity of an absorbent layer 120, a moisture level within the wound dressing 100, etc.) are each similar, or substantially similar, to one another. Alternatively (or additionally), in other embodiments, one or more of the plurality of indicators 10 used to monitor a particular type of fluid status may be defined by varying characteristics and properties. For example, the plurality of indicators 10 used to monitor a particular type of fluid status optionally comprise at least a first indicator 10 and a second indicator 10 that are configured to transition in response to varying fluid level thresholds, and thus may be used to provide a user with an even more granular and dynamic ability to monitor fluid status. For example, in some embodiments a first indicator 10 may be defined by a transition threshold such that a marking 25 (e.g. a red-colored indicia) becomes visible upon the first indicator 10 being exposed to a fluid level corresponding to a maximum capacity of the absorbent layer 120. A second indicator 10 in such an embodiment may be defined by a transition threshold such that a marking 25 thereon (e.g. an orange-colored indicia) becomes visible upon the second indicator 10 being exposed to a fluid level corresponding to an different fluid capacity of the absorbent layer 120, and which may thus provide a viewer with additional notice that the absorbent layer 120 may be reaching its absorbency capacity.

Given the different types of fluid level changes it may be desirable to monitor during the treatment of a wound 115 (e.g., a remaining fluid capacity of an absorbent layer 120, moisture at a periwound, progress of wound healing, etc.), in some embodiments, a plurality of indicators 10 may alternatively (or additionally) be incorporated into the wound dressing 100 to monitor a variety of different types of fluid statuses associated with the use of the wound dressing 100. For example, a wound dressing 100 may optionally include both a first indicator 10 positioned along an outer perimeter of the wound dressing 100 that is configured to signal the presence of fluid at a periwound, as well as a second indicator 10 positioned atop an absorbent layer 120 that is configured to alert a user when the absorbent layer 120 of the wound dressing 100 nears (or reaches) its maximum fluid capacity.

In addition to varying the size, shape and color of the markings 25, the ability to distinguish whether the wound dressing 100 component and portion of the wound 115 being monitored has been exposed to fluids and has transitioned to the wet state is optionally aided by the incorporation of optional outlines (or indicia) along those portions of the top layer 40 and/or drape layer 110 that overlie each markings 25. The outlines are visible in each of the dry state and the wet state, and encircle or otherwise visually draw attention to those portions of the wound dressing 100 at which a view may expect to view a marking 25. Such outlines may be used with both embodiments comprising a single indicator 10 as well as those embodiments comprising a plurality of indicators 10.

### Configuration of Exemplary Embodiments

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the disclosure as recited in the appended claims.

It should be noted that the term "exemplary" and variations thereof, as used herein to describe various embodiments, are intended to indicate that such embodiments are possible examples, representations, or illustrations of possible embodiments (and such terms are not intended to connote that such embodiments are necessarily extraordinary or superlative examples).

The term "coupled" and variations thereof, as used herein, means the joining of two members directly or indirectly to one another. Such joining may be stationary (e.g., permanent or fixed) or moveable (e.g., removable or releasable). Such joining may be achieved with the two members coupled directly to each other, with the two members coupled to each other using a separate intervening member and any additional intermediate members coupled with one another, or with the two members coupled to each other using an intervening member that is integrally formed as a single unitary body with one of the two members. If "coupled" or variations thereof are modified by an additional term (e.g., directly coupled), the generic definition of "coupled" provided above is modified by the plain language meaning of the additional term (e.g., "directly coupled" means the joining of two members without any separate intervening member), resulting in a narrower definition than the generic definition of "coupled" provided above. Such coupling may be mechanical, electrical, or fluidic.

The term "or," as used herein, is used in its inclusive sense (and not in its exclusive sense) so that when used to connect a list of elements, the term "or" means one, some, or all of the elements in the list. Conjunctive language such as the phrase "at least one of X, Y, and Z," unless specifically stated otherwise, is understood to convey that an element may be either X, Y, Z; X and Y; X and Z; Y and Z; or X, Y, and Z (i.e., any combination of X, Y, and Z). Thus, such conjunctive language is not generally intended to imply that certain embodiments require at least one of X, at least one of Y, and at least one of Z to each be present, unless otherwise indicated.

References herein to the positions of elements (e.g., "top," "bottom," "above," "below") are merely used to describe the orientation of various elements in the FIGURES. It should be noted that the orientation of various elements may differ according to other exemplary embodiments, and that such variations are intended to be encompassed by the present disclosure.

## Claims

1. An indicator for detecting a fluid status of a wound dressing, the indicator comprising:
a lower layer (20);
a marking (25) provided along the lower layer (20); and
a top layer (40);
wherein in a first state, the marking (25) is blocked by the top layer (40) and not visible to a user under ambient or natural light;
wherein in a second state, the marking (25) is visible through the top layer (40) under ambient or natural light;
wherein in a third state, the marking (25) is blocked by the top layer (40) and not visible to a user under ambient or natural light, the third state occurring at a point in time subsequent to the second state;
wherein the top layer (40) is formed from a porous, hydrophilic structure defined by a plurality of fibers; and
wherein, in the first state, the fibers have a first refractive index and in the second state the fibers have a second refractive index that is greater than the first refractive index.

2. The indicator of claim 1, wherein the first state is representative of an absence of absorbed wound fluid, the second state is representative of a presence of absorbed wound fluid, and the third state is representative of a reduction by evaporation of the wound fluid present in the second state.

3. The indicator of claim 1, wherein the indicator is exposed to a fluid in the second state, the material from which the fibers are formed being selected such that, when exposed to the fluid, the second refractive index of the fibers is substantially the same as the refractive index of the fluid.

4. The indicator of claim 3, wherein the second refractive index of the fibers is between approximately 1.30 and approximately 1.35.

5. The indicator of claim 1, further comprising an attachment layer (30) extending between and attaching the top layer (40) and the lower layer (20).

6. The indicator of claim 1, wherein the marking (25) is configured to stay fixed relative to the lower layer (20) when the lower layer (20) is exposed to a fluid.

7. A wound treatment system, comprising:
a negative pressure source;
a wound dressing (100) configured to be attached to a patient;
a conduit configured to fluidly couple the negative pressure source to a treatment space defined underneath the wound dressing upon application of the wound dressing to the patient; and
an indicator according to any preceding claim.

8. The wound treatment system of claim 7, wherein the wound dressing comprises:
a manifolding layer (130) configured to distribute negative pressure generated by the negative pressure source to a wound located beneath the wound dressing; and
a drape layer (110) configured to secure the manifolding layer (130) relative to the patient;
wherein a lower surface of the indicator directly contacts the manifolding layer (130).

9. The wound treatment system of claim 7, wherein the wound dressing comprises:
a manifolding layer (130) configured to distribute negative pressure generated by the negative pressure source to a wound located beneath the wound dressing; and
a drape layer (110)configured to secure the manifolding layer (130) relative to the patient, an outer perimeter of the drape layer (110) defining a border that extends outwards relative to an outer perimeter of the manifolding layer (130) and is configured to be secured to the patient;
wherein the indicator is positioned at a location along the border defined by the drape layer (110).

10. The wound treatment system of claim 9, wherein an upper surface of the indicator directly contacts a lower surface of the drape layer (110) and a lower surface of the indicator directly contacts a portion of the skin of the patient surrounding the wound upon application of the wound dressing to the patient.

11. The wound treatment system of claim 9, wherein an upper surface of the indicator directly contacts to a lower surface of the drape layer (110) and a lower surface of the indicator directly contacts an upper surface of a wound interface layer.

12. The wound treatment system of claim 7, further comprising a connector (250) configured to be sealed around an opening formed in a drape layer (110) of the wound dressing to fluidly couple the negative pressure source to the wound dressing, wherein the indicator is positioned between an upper surface of the drape layer (110) and a lower surface of the connector (250).

## Patentansprüche

1. Ein Indikator zum Erfassen eines Fluidstatus eines Wundverbands, der Indikator aufweisend: eine untere Schicht (20);
eine Markierung (25),
die entlang der unteren Schicht (20) bereitgestellt ist; und
eine obere Schicht (40);
wobei, in einem ersten Zustand, die Markierung (25) mittels der oberen Schicht (40) blockiert und für einen Benutzer unter Umgebungs- oder natürlichem Licht nicht sichtbar ist;
wobei, in einem zweiten Zustand, die Markierung (25) durch die obere Schicht (40) unter Umgebungs- oder natürlichem Licht sichtbar ist;
wobei, in einem dritten Zustand, die Markierung (25) mittels der oberen Schicht (40) blockiert und für einen Benutzer unter Umgebungs- oder natürlichem Licht nicht sichtbar ist, wobei der dritte Zustand zu einem Zeitpunkt im Anschluss an den zweiten Zustand auftritt;
wobei die obere Schicht (40) aus einer porösen hydrophilen Struktur, die mittels einer Vielzahl von Fasern definiert ist, ausgebildet ist; und
wobei, in dem ersten Zustand, die Fasern einen ersten Brechungsindex haben und, in dem zweiten Zustand, die Fasern einen zweiten Brechungsindex, der größer als der erste Brechungsindex ist, haben.

2. Der Indikator nach Anspruch 1, wobei der erste Zustand für ein Nichtvorhandensein von absorbiertem Wundfluid repräsentativ ist, der zweite Zustand für ein Vorhandensein von absorbiertem Wundfluid repräsentativ ist und der dritte Zustand für eine Verringerung mittels Verdampfung des Wundfluids, das in dem zweiten Zustand vorhanden ist, repräsentativ ist.

3. Der Indikator nach Anspruch 1, wobei der Indikator einem Fluid in dem zweiten Zustand ausgesetzt ist, wobei das Material, aus dem die Fasern ausgebildet sind, derart ausgewählt wird, dass, wenn es dem Fluid ausgesetzt wird, der zweite Brechungsindex der Fasern im Wesentlichen der gleiche wie der Brechungsindex des Fluids ist.

4. Der Indikator nach Anspruch 3, wobei der zweite Brechungsindex der Fasern zwischen etwa 1,30 und etwa 1,35 liegt.

5. Der Indikator nach Anspruch 1, ferner aufweisend eine Befestigungsschicht (30), die sich zwischen der oberen Schicht (40) und der unteren Schicht (20) erstreckt und diese befestigt.

6. Der Indikator nach Anspruch 1, wobei die Markierung (25) konfiguriert ist, um bezüglich der unteren Schicht (20) fixiert zu bleiben, wenn die untere Schicht (20) einem Fluid ausgesetzt ist.

7. Ein Wundbehandlungssystem, aufweisend:
eine Unterdruckquelle;
einen Wundverband (100), der konfiguriert ist, um an einem Patienten befestigt zu werden;
eine Leitung, die konfiguriert ist, um die Unterdruckquelle mit einem Behandlungsraum, der unterhalb des Wundverbands bei einer Applikation des Wundverbands an dem Patienten definiert wird, fluidisch zu koppeln; und
einen Indikator nach einem der vorstehenden Ansprüche.

8. Das Wundbehandlungssystem nach Anspruch 7, wobei der Wundverband aufweist:
eine Verteilungsschicht (130), die konfiguriert ist, um einen Unterdruck, der mittels der Unterdruckquelle erzeugt wird, auf eine Wunde, die sich unter dem Wundverband befindet, zu distribuieren; und
eine Abdecktuchschicht (110), die konfiguriert ist, um die Verteilungsschicht (130) bezüglich des Patienten anzubringen;
wobei eine untere Oberfläche des Indikators die Verteilungsschicht (130) direkt berührt.

9. Das Wundbehandlungssystem nach Anspruch 7, wobei der Wundverband aufweist:
eine Verteilungsschicht (130), die konfiguriert ist, um einen Unterdruck, der mittels der Unterdruckquelle erzeugt wird, auf eine Wunde, die sich unter dem Wundverband befindet, zu distribuieren; und
eine Abdecktuchschicht (110), die konfiguriert ist, um die Verteilungsschicht (130) bezüglich des Patienten anzubringen, wobei ein Außenumfang der Abdecktuchschicht (110) eine Grenze definiert, die sich bezüglich eines Außenumfangs der Verteilungsschicht (130) nach außen erstreckt und konfiguriert ist, um an dem Patienten angebracht zu werden;
wobei der Indikator an einer Stelle entlang der Grenze, die mittels der Abdecktuchschicht (110) definiert wird, positioniert ist.

10. Das Wundbehandlungssystem nach Anspruch 9, wobei eine obere Oberfläche des Indikators eine untere Oberfläche der Abdecktuchschicht (110) direkt berührt und eine untere Oberfläche des Indikators einen Abschnitt der Haut des Patienten, der die Wunde bei der Applikation des Wundverbands an dem Patienten umgibt, direkt berührt.

11. Das Wundbehandlungssystem nach Anspruch 9, wobei eine obere Oberfläche des Indikators eine untere Oberfläche der Abdecktuchschicht (110) direkt berührt und eine untere Oberfläche des Indikators eine obere Oberfläche einer Wundzwischenschicht direkt berührt.

12. Das Wundbehandlungssystem nach Anspruch 7, ferner aufweisend einen Verbinder (250), der konfiguriert ist, um um eine Öffnung herum, die in einer Abdecktuchschicht (110) des Wundverbands ausgebildet ist, abgedichtet zu sein, um die Unterdruckquelle mit dem Wundverband fluidisch zu koppeln, wobei der Indikator zwischen einer oberen Oberfläche der Abdecktuchschicht (110) und einer unteren Oberfläche des Verbinders (250) positioniert ist.

## Revendications

1. Indicateur permettant de détecter un état de fluide d'un pansement pour plaie, l'indicateur comprenant :
une couche inférieure (20) ;
un repère (25) prévu le long de la couche inférieure (20) ; et
une couche supérieure (40) ;
dans lequel, dans un premier état, le repère (25) est bloqué par la couche supérieure (40) et invisible par un utilisateur sous lumière ambiante ou naturelle ;
dans lequel, dans un deuxième état, le repère (25) est visible à travers la couche supérieure (40) sous une lumière ambiante ou naturelle ;
dans lequel dans un troisième état, le repère (25) est bloqué par la couche supérieure (40) et invisible par un utilisateur sous lumière ambiante ou naturelle, le troisième état se produisant à un moment postérieur au deuxième état ;
dans lequel la couche supérieure (40) est formée à partir d'une structure hydrophile poreuse définie par une pluralité de fibres ; et
dans lequel, dans le premier état, les fibres ont un premier indice de réfraction, et dans le deuxième état, les fibres ont un second indice de réfraction qui est supérieur au premier indice de réfraction.

2. Indicateur selon la revendication 1, dans lequel le premier état est représentatif d'une absence de fluide de plaie absorbé, le deuxième état est représentatif d'une présence de fluide de plaie absorbé, et le troisième état est représentatif d'une réduction par évaporation du fluide de plaie présent dans le deuxième état.

3. Indicateur selon la revendication 1, dans lequel l'indicateur est exposé à un fluide dans le deuxième état, le matériau à partir duquel les fibres sont formées étant choisi de telle sorte que, lorsqu'il est exposé au fluide, le deuxième indice de réfraction des fibres est sensiblement identique à l'indice de réfraction du fluide.

4. Indicateur selon la revendication 3, dans lequel le deuxième indice de réfraction des fibres est compris entre environ 1,30 et environ 1,35.

5. Indicateur selon la revendication 1, comprenant en outre une couche de fixation (30) s'étendant entre la couche supérieure (40) et la couche inférieure (20) et fixant celles-ci.

6. Indicateur selon la revendication 1, dans lequel le repère (25) est conçu pour rester fixe par rapport à la couche inférieure (20) lorsque la couche inférieure (20) est exposée à un fluide.

7. Système de traitement de plaie, comprenant :
une source de pression négative ;
un pansement pour plaie (100) conçu pour être fixé à un patient ;
un conduit conçu pour accoupler fluidiquement la source de pression négative à un espace de traitement défini en dessous du pansement pour plaie lors de l'application du pansement pour plaie au patient ; et
un indicateur selon l'une quelconque revendication précédente.

8. Système de traitement de plaie selon la revendication 7, dans lequel le pansement pour plaie comprend :
une couche de collecte (130) conçue pour distribuer une pression négative générée par la source de pression négative à une plaie située sous le pansement pour plaie ; et
une couche de nappe (110) conçue pour fixer la couche de collecte (130) par rapport au patient ;
dans lequel une surface inférieure de l'indicateur est directement en contact avec la couche de collecte (130).

9. Système de traitement de plaie selon la revendication 7, dans lequel le pansement pour plaie comprend :
une couche de collecte (130) conçue pour distribuer une pression négative générée par la source de pression négative à une plaie située sous le pansement pour plaie ; et
une couche de nappe (110) conçue pour fixer la couche de collecte (130) par rapport au patient, un périmètre externe de la couche de nappe (110) définissant une bordure qui s'étend vers l'extérieur par rapport à un périmètre externe de la couche de collecte (130) et est conçue pour être fixée au patient ;
dans lequel l'indicateur est positionné à un emplacement le long de la bordure définie par la couche de nappe (110).

10. Système de traitement de plaie selon la revendication 9, dans lequel une surface supérieure de l'indicateur est directement en contact avec une surface inférieure de la couche de nappe (110) et une surface inférieure de l'indicateur est directement en contact avec une partie de la peau du patient entourant la plaie lors de l'application du pansement pour plaie au patient.

11. Système de traitement de plaies selon la revendication 9, dans lequel une surface supérieure de l'indicateur est directement en contact avec une surface inférieure de la couche de nappe (110) et une surface inférieure de l'indicateur est directement en contact avec une surface supérieure d'une couche d'interface de plaie.

12. Système de traitement de plaie selon la revendication 7, comprenant en outre un connecteur (250) conçu pour être scellé autour d'une ouverture formée dans une couche de nappe (110) du pansement pour plaie pour accoupler fluidiquement la source de pression négative au pansement pour plaie, dans lequel l'indicateur est positionné entre une surface supérieure de la couche de nappe (110) et une surface inférieure du connecteur (250).
